Europäisches Patentamt

European Patent Office

Office européen des brevets

(11) Publication number: **0 260 715**
**A2**

## (12) EUROPEAN PATENT APPLICATION

(21) Application number: **87113710.5**

(22) Date of filing: **18.09.87**

(51) Int. Cl.⁴: **C12P 21/00** , G01N 33/577 , G01N 33/574 , C12N 5/00

(30) Priority: **18.09.86 JP 220049/86**

(43) Date of publication of application:
**23.03.88 Bulletin 88/12**

(84) Designated Contracting States:
**DE FR GB**

(71) Applicant: **Shiku, Hiroshi**
**10-16, Shiratori-machi**
**Nagasaki-shi Nagasaki(JP)**

(72) Inventor: **Shiku, Hiroshi**
**10-16, Shiratori-machi**
**Nagasaki-shi Nagasaki(JP)**

(74) Representative: **Kinzebach, Werner, Dr.**
**Patentanwälte Reitstötter, Kinzebach &**
**Partner Sternwartstrasse 4 Postfach 86 06 49**
**D-8000 München 86(DE)**

(54) **Monoclonal antibody.**

(57) Monoclonal antibodies produced by fusion cells (hybridomas) between spleen cells of mice immunized with the Ki-ras oncogene product and mouse myeloma cells react with various cancer cells and can be utilized in the diagnosis of cancer.

EP 0 260 715 A2

Xerox Copy Centre

## MONOCLONAL ANTIBODY

FIELD OF THE INVENTION

This invention relates to monoclonal antibodies to the Ki-ras oncogene product, hybridomas capable of producing said antibodies, and a method of cancer detection which uses said antibodies. Accordingly, the invention is of use in the area of clinical cancer diagnosis.

BACKGROUND OF THE INVENTION

The role of oncogenes in oncogenesis of human cells is currently worthy of attention. Known oncogenes include ras genes and myc genes, among others, the attempts have been made to detect cancer using, as markers, products encoded by these genes.

As regards the ras gene products, a method of detecting cancer which uses monoclonal antibodies to said products is known. The ras genes include such species as Ha-ras, Ki-ras and N-ras, and the products encoded by these ras genes are proteins having a molecular weight of 21,000 and designated as Ha-p21, Ki-p21 and N-p21, respectively (hereinafter collectively referred to as "p21").

As monoclonal antibodies to p21, there are known rat monoclonal antibodies to p21 produced by using, as antigen, rat kidney-derived NRK (normal rat kidney) cells transformed with Harvey murine sarcoma virus (J. Virol., 43, 294-304 (1982)) and mouse monoclonal antibodies produced using, as antigen, a synthetic peptide corresponding to the 10th (from the N terminus) to 17th amino acid residues of the Ha-ras gene product (Proc. Natl. Acad. Sci., USA, 81, 5227-5231 (1984)), for instance.

Monoclonal antibodies as produced with N-ras or Ki-ras gene products as antigen are not known, however.

Attempts to use, in cancer diagnosis, existing monoclonal antibodies that react with the Ha-ras gene product have already been made. For instance, antibodies to the Ha-ras gene product that give positive results in the immunohistological detection of human colon, breast, prostate and stomach carcinomas are known. However, the range of carcinomas detectable with such monoclonal antibodies is limited. Better monoclonal antibodies are needed which have an affinitiy for additional types of cancers.

SUMMARY OF THE INVENTION

It has now been found that monoclonal antibodies, produced by the fusion cells (hybridomas) between spleen cells of mice immunized with the Ki-ras gene product and mouse myeloma cells, react with various cancer cells and therefore can be utilized in the diagnosis of cancer. These monoclonal antibodies are reactive particularly with human thyroid carcinoma and are very useful in cancer diagnosis since, heretofore, no monoclonal antibodies have been known to be effective in detecting human thyroid carcinoma.

DETAILED DESCRIPTION OF THE INVENTION

Monoclonal antibodies according to the invention, a method of producing the same and a method of detecting cancer using the same all according to the present invention are now described in detail.

First of all, the invention provided monoclonal antibodies capable of reacting with the Ki-ras gene product. Monoclonal antibodies according to the invention can be produced by immunizing mice with the Ki-ras gene product, fusing the mouse spleen cells (or splenocytes) thus obtained with mouse myeloma cells, selecting, from among the fused cells (hybridomas) obtained, cells capable of reacting with the Ki-ras gene product and cultivating these cells.

Hybridoma production may be performed by the method of Kato et al (GANN, 76, 524-531 (1985)), which is a modification of the method of Köhler and Milstein (Nature, 256, 495-497 (1975)).

As the Ki-ras gene product, Ki-p21 produced using the Kirstein sarcoma virus-derived Ki-ras oncogene and the recombinant DNA technique of Tamaoki et al. (Biochem. Biophys. Res. Comm., 132, 126-133 (1985)) is preferably used, while any Ki-ras gene product obtained by other methods can also be used.

2

The mice to be immunized are , for example, BALB/c mice or F1 mice from BALB/c mice and mice of another strain.

Immunization is carried out 3-6 times at 2-to 3-week intervals using 50-200 μg of protein per mouse (6-8 weeks of age, 20-30 grams). Mouse breeding and splenocyte collection are conducted in the conventional manner.

Suitable myeloma cell lines include MOPC-21 NS/1 (Nature, 256, 495-497 (1975)), SP2/0-Ag14 (Nature, 277, 131-133 (1979)) and S194/5, XXO.BU.1 (J. Exp. Med., 148, 313-328 (1978)), among others. Splenocytes and myeloma cells are mixed together at a ratio of 1:1 to 10:1 and fusion is carried out in a phosphate buffer (pH 7.2-7.4) containing NaCl (about 0.85%), dimethyl sulfoxide (10-20% (v/v)) and polyethylene glycol having a molecular weight of 1,000-6,000.

Cell fusion is effected by incubating the mixture of both kinds of cells at 35-37°C for 1-3 minutes.

Selection of fused cells (hybridomas) is carried out using a basal medium containing hypoxanthine (1.3-1.4 mg/dl), aminopterine (18-20 μg/dl), thymidine (375-4,000 μg/dl),streptomycin (50-100 μg/ml), penicillin (50-100 units/ml), glutamine (3.5-4.0 g/liter) and fetal calf serum (10-20%), and cells that grow are selected.

Any of those media which are generally used in animal cell culture, for example RPMI 1640 medium and Eagle's MEM medium, may be used as the basal medium.

Cloning of hybridomas is repeated at least three times by limiting dilution.

Cultivation of the hybridomas, which is carried out in the same manner as the conventional animal cell culture, leads to formation of the antibodies according to the invention in the medium. Thus, for instance, growing $2 \times 10^6$ to $5 \times 10^6$ hybridoma cells in 10-20 ml of RPMI 1640 medium containing streptomycin (50-100 μg/ml), penicillin (50-100 units/ml), glutamine 3.5-4.0 g/liter) and fetal calf serum (10-20%) in a flask in the presence of 5% $CO_2$ at 35-37°C for 3-7 days results in antibody secretion and accumulation in the culture.

It is also possible to transplant hybridoma cells into the abdominal cavity of pristane-treated nude mice or BALB/c mice and allow said cells to grow there to thereby cause accumulation of the antibodies according to the invention in the ascitic fluid. Thus, such mice are injected with 0.5-1 ml of pristane (2,6,10,14-tetramethylpentadecane, Aldrich, USA) and, 2-3 weeks later, $5 \times 10^6$ to $10 \times 10^6$ hybridoma cells are transplanted into their abdominal cavity. Generally, 7-10 days thereafter, the ascitic fluid is found accumulated and is collected.

The monoclonal antibodies in the culture or ascitic fluid are isolated by affinity chromatography using Affi-Gel Protein A MAPS-II® Kit (Nippon Bio-rad Laboratories).

Among the monoclonal antibodies thus obtained, there may be mentioned those designated as RASK-1, RASK-2, RASK-3 and RASK-4 are typical examples. These are characterized by the following physico-chemical and biological properties as shown in Table 1.

## TABLE 1

|  | RASK-1 | RASK-2 | RASK-3 | RASK-4 |
|---|---|---|---|---|
| Subtype | IgG1 | IgG1 | IgG2b | IgG2b |
| Reactivity | Specific to Ki-ras p21 | Strongly reactive with Ki-ras p21 | Reactive with Ki-, N- and Ha-ras p21 | Reactive with Ki-, N- and Ha-ras p21 |

The hybridomas capable of producing said monoclonal antibodies according to the invention have been deposited under the Budapest Treaty with the European Collection of Animal Cell Cultures in England since September 11, 1986 under the deposit numbers ECACC 86091101, 86091102, 86091103, and 86091104, respectively.

The biological activities of the monoclonal antibodies according to the invention are as shown in Examples 2-7. The procedures followed in the examples for indirect immunofluorescence assay (IF assay), enzyme-linked immunosorbent assay (ELISA), immunoblotting and avidinbiotin complex enzyme immunoassay (ABC assay) are as follows. In these assays, monoclonal antibodies according to the present invention are used as a solution of phosphate-buffered physiological saline(hereinafter referred to as "PBS")in a concentration of about 20 μg/ml.

Indirect Immunofluorescence Assay (IF Assay):

Cultured human cancer cells suspended in MEM medium supplemented with 10% fetal calf serum ($10^5$ cells/ml) are distributed into the wells of a 60-well microplate and cultured for 24 hours at 37°C in the presence of 5% $CO_2$, whereby they are allowed to adhere to the plate. After washing with PBS cells are fixed by treatment with 10% formalin at room temperature for 60 minutes and then again washed with PBS. A monoclonal antibody solution is distributed in 10-μl portions into the wells. After 45 minutes of incubation at room temperature, cells are washed, and 10 μl of a fluorochrome-labeled goat anti-mouse immunoglobulin antibody (Coulter) was added to each well. After 30 minutes of reaction at room temperature, cells are washed again with PBS and examined under a fluorescence microscope for fluorescence emission therefrom.

Enzyme-linked Immunosorbent Assay (ELISA) :

A solution of the target ras-p21 protein (50 μg/ml) is distributed in 50-μl portions into the wells of a 96-well immunoplate for ELISA. After allowing to stand at 4°C for 24 hours and subsequent washing with 0.5% Tween 20-supplemented PBS (hereinafter the same being used for washing unless otherwise specified), blocking is carried out with 100 μl (per well) of a 5% bovine serum albumin solution at room temperature for 1 hour. After washing, 50 μl of an appropriately diluted monoclonal antibody solution is added to each well and the reaction is allowed to proceed at room temperature for 45 minutes. After washing again, 50 μl of a 2,000-fold dilution of a peroxidase-labeled goat anti-mouse immunoglobulin antibody (Igaku Seibutsugaku Kenkyusho) is added to each well. After 45 minutes of reaction at room temperature and subsequent washing, color development is caused by using o-phenylenediamine as the substrate and the absorbance is measured at a wavelength of 492 nm on an automatic ELISA reader (Labo-Science model SLT210).

Avidin-Biotin Complex Enzyme Immunoassay (ABC Method):

A 4-μm-thick section is sliced off from each paraffin-embedded, 10% formalin-fixed tissue preparation, caused to adhere to a slide and deparaffinized. After washing with distilled water, the section is treated with 100% ethanol containing 0.3% hydrogen peroxide for 30 minutes and then washed three times with distilled water. Normal horse serum (Vectastain® ABC Kit, Vector Laboratories) is added dropwise onto the section, followed by incubation for 20 minutes. Thereafter, an appropriately diluted monoclonal antibody solution is added and incubation is carried out at room temperature for 30 minutes. After washing with three portions of PBS, boitinylated goat anti-mouse immunoglobulin antibody (Vectastain® ABC Kit, Vector Laboratories) is added, followed by 30 minutes of reaction. After washing three times, avidin-biotin peroxidase complex (Vectastain® ABC Kit, Vector Laboratories) is added and the reaction is allowed to proceed for 1 hour. After washing again three times, the section is treated with 0.05 M Tris-hydrochloride buffer (pH 7.2) containing 0.05% diaminobenzidine and 0.01% hydrogen peroxide at room temperature for 5 minutes. After washing with water, hematoxyline staining is performed in the conventional manner and the section is examined under a microscope for substrate coloration in the tissue.

Immunoblotting (Towin, H. et al., Proc. Natl. Acad. Sci. USA, 76, 4350 (1979)) :

Each sample is subjected to SDS-polyacrylamide electrophoresis, and peptides (ras oncogene-encoded p21 protein, etc.) are electrically transferred to a nitrocellulose sheet and then reacted with a monoclonal antibody. After washing, the reaction with biotinylated anti-mouse immunoglobulin antibody (Vectastain® ABC Kit, Vector Laboratories) is carried out and, after washing again, the reaction with avidin-biotin peroxidase complex (Vectastain® ABC Kit, Vector Laboratories) is conducted, followed by color development using 4-chloro-1-naphtol as the substrate. The molecular weight of each reactive protein can be measured based on the index of reaction of the monoclonal antibody with a specific protein (the p21 protein in the present case) or the electrophoretic mobility (distance of travel).

Monoclonal antibodies according to the invention are reactive with various cancer cells and cancer tissues. For instance, they are reactive with human thyroid carcinoma, human stomach carcinoma, human breast carcinoma, human uterine carcinoma, human colon carcinoma, and so forth. Previously there were no monoclonal antibodies known to be reactive with human thyroid carcinoma. Accordingly, the monoclonal antibodies according to the invention are particularly useful in detecting thyroid carcinoma. For the purpose of cancer diagnosis, they are useful in tissue staining and serodiagnosis such as mentioned above. Some examples of their application are shown hereinbelow in the Examples.

## EXAMPLE 1

Production of Monoclonal Antibodies, RASK-1, RASK-2, RASK-3 and RASK-4:

The Ki-ras oncogene-encoded protein (Ki-p21) was produced by the method of Tamaoki et al. (Biochem. Biophys. Res. Comm. , 132, 126-133 (1985)) using the Kirstein sarcoma virus-derived Ki-ras oncogene.

Ki-p21 (50 $\mu$g as protein) was mixed with 0.02 ml of complete Freund's adjuvant (Difco), and a (BALB/c $\times$ C57BL/6) F1 mouse (8 weeks of age, 25 g; purchased from Shizuoka Laboratory Animal Center) was immunized with the mixture by subcutaneous inoculation at the back. Two weeks later, the same quantity of the Ki-p21 protein was subcutaneously inoculated, in admixture with 0.02 ml of incomplete Freund's adjuvant (Difco), into the same mouse at the back thereof. After an additional two weeks and four weeks, 100 $\mu$g and 200 $\mu$g of the protein alone were intraperitoneally inoculated into the mouse, respectively. Four days after the final immunization, the mouse was sacrificed, and the spleen was excised and treated in the conventional manner to give a cell suspension.

Splenocytes ($10^8$ cells) from the suspension were subjected to fusion with $2 \times 10^7$ mouse myeloma MOPC-21NS/1 cells (Nature , 256, 495-497 (1975)) in 0.2 ml of phosphate-buffered physiological saline (PBS) containing 42% (w/v) polyethylene glycol (mol. wt. 4,000, Koch-Light, Bucks, UK) and 15% (v/v) dimethyl sulfoxide (Merck, Darmstadt, West Germany) by the method of Kato et al. (GANN, 76, 524-531 (1985)).

Selection of fused cells was performed by the above-mentioned method of Kato et al. using HAT medium (RPMI 1640 medium (pH 7.2) containing 1.36 mg/dl hypoxanthine, 19.1 $\mu$g/dl aminopterine, 387 $\mu$g/dl thymidine, 100 $\mu$g/ml streptomycin, 100 units/ml penicilline, 2mM glutamine and 10% fetal calf serum).

Four fused cells thus obtained were cloned by two repetitions of limiting dilution. The four clones were cultured by the method mentioned below, whereby four cell lines each capable of producing a monoclonal antibody specifically reactive with the Ki-p21 protein antigen in large quantities were obtained. Thus, $5 \times 10^6$ cells of each clone were cultured in 15 ml of RPMI 1640 medium containing 100 $\mu$g/ml streptomycin, 100 units/ml penicilline, 3.8 g/liter glutamine and 10% fetal calf serum in a 150-ml flask in the presence of 95% $O_2$-5% $CO_2$ at 37°C for 7 days.

The monoclonal antibodies produced by these four clones were designated RASK-1, RASK-2, RASK-3 and RASK-4, respectively. (BALB/c $\times$ C57BL/6) F1 mouse (purchased from Shizuoka Laboratory Animal Center) preliminarily injected with 0.5 ml of pristane were intraperitoneally inoculated with $5 \times 10^6$ cells of one of the clones. After 2 weeks to 1 month, about 1-5 liters of ascitic fluid was collected. Affinity chromatography of the ascitic fluid using Affi-Gel Protein A MAPS-II® Kit (Nippon Bio-Rad Laboratories) gave the monoclonal antibody RASK-1, RASK-2, RASK-3 or RASK-4 in a yield of about 2-10 mg per milliliter of ascitic fluid.

## EXAMPLE 2

Reactivity Features of the Monoclonal Antibodies Relative to Various Cultured Cell Lines:

The monoclonal antibodies obtained in Example 1 were examined for their reactivity with cytoplasmic antigens of various culture cell lines using the IF assay technique mentioned above. The cell lines used were Calu1 (lung carcinoma), Paca2 (pancreatic carcinoma), SK-NSH (neuroblastoma), SW13 (adrenocortical carcinoma) and HL60 (promyelocytic leukemia).

The monoclonal antibodies according to the invention, namely RASK-1, RASK-2, RASK-3 and RASK-4, all showed reactivity with all the cell lines mentioned above.

The degrees of reaction were as shown below in Table 2.

### TABLE 2

| Sample | RASK-1 | RASK-2 | RASK-3 | RASK-4 |
|--------|--------|--------|--------|--------|
| Calu-1 | + | + | ++ | ++ |
| Paca 2 | + | + | ++ | ++ |
| SK-NSH | + | + | ++ | ++ |
| SW13 | + | + | ++ | ++ |
| HL60 | + | + | ++ | ++ |

## EXAMPLE 3

Reactivity of the Monoclonal Antibodies with ras Oncogene-Encoded p21 Proteins:

The monoclonal antibodies obtained in Example 1 were examined for their reactivity with ras oncogene-encoded p21 proteins by the above-mentioned ELISA and immunoblotting techniques.

In ELISA, in which the Ki-p21 protein used for immunonization was used as the solid phase, all the antibodies RASK-1, RASK-2, RASK-3 and RASK-4 showed strong positive reaction. The antibody titers of these antibodies to Ki-p21 as found in ELISA were as follows: RASK-1, 1/1,000; RASK-2, 1/4,000; RASK-3, 1/4,000; and RASK-4, 1/4,000. In ELSA, in which the N-ras oncogene-encoded protein N-p21 was used as the solid phase, RASK-3 and RASK-4 showed very strong positive reaction while RASK-1 showed negative reaction and RASK-2 very weak positive reaction.

The reactivities of these antibodies with ras oncogene-encoded p21 proteins were examined by the immunoblotting technique. Thus, immunoblotting was performed using the Ki-p21 and N-p21 proteins as well as a Harvey sarcoma virus-transformed cell extract and a Kirstein sarcoma virus-transformed cell extract. The results obtained, which shown in Table 3, indicate that RASK-1 is reactive with the Ki-ras oncogene-derived p21 (Ki-p21) but is not reactive with the N-ras oncogene-derived p21 (N-p21) or Harvey sarcoma virus-transformed cells, that RASK-2 is reactive with the Ki-ras oncogene-derived p21 protein (Ki-p21), very weakly reactive with the N-ras oncogene-derived p21 protein (N-p21) and quite inert to Harvey sarcoma virus-transformed cells, and that RASK-3 and RASK-4 are reactive with all the p21 proteins, namely the Ki-ras oncogene-, N-ras oncogene-and Ha-ras oncogene-derived p21 proteins.

## TABLE 3

| Mono-clonal Antibody | Reactivity with p21 Protein | | | |
|---|---|---|---|---|
| | RASK-1 | RASK-2 | RASK-3 | RASK-4 |
| RASK-1 | + | − | − | + |
| RASK-2 | + | − | − | + |
| RASK-3 | + | + | + | + |
| RASK-4 | + | + | + | + |

### EXAMPLE 4

Whether the monoclonal antibodies according to the invention have the same or different combining sites for the Ki-ras protein was investigated using biotinylated RASK-3 and RASK-4.

The monoclonal antibodies were biotinylated by incubating RASK-3 and RASK-4 (obtained by using Affi-Gel Protein A MAPS-II®, as mentioned above) with N-hydroxy-succinimide-biotin at room a temperature for 4 hours.

The Ki-ras protein p21 was coupled to a plate for ELISA, then RASK-1, RASK-2, RASK-3 and RASK-4 were added and preliminary incubation was performed at room temperature for 1 hour. Thereafter, 1/2,000, 1/4,000 and 1/8,000 dilutions of biotinylated RASK-3 or RASK-4 were added, and ELISA was performed by the ABC method. The results thus obtained are shown in Table 4. They indicate that the binding of biotinylated RASK-3 to Ki-ras p21 is inhibited by RASKs in the order of RASK-3 > RASK-4 > RASK-2 > RASK-1. On the other hand, the binding of biotinylated RASK-4 to Ki-ras p21 is inhibited by RASK-1. These results suggest that RASK-3 and RASK-4 react with the same p21 at different sites thereof.

## TABLE 4

| Inhi-biting Antibody | Biotinylated Antibody | | | | | |
|---|---|---|---|---|---|---|
| | RASK-3 | | | RASK-4 | | |
| | 1/2000 | 1/4000 | 1/8000 | 1/2000 | 1/4000 | 1/8000 |
| RASK-1 | 0.60 | 0.30 | 0.11 | 0.66 | 0.40 | 0.16 |
| RASK-2 | 0.38 | 0.17 | 0.06 | 0.53 | 0.31 | 0.12 |
| RASK-3 | 0 | 0 | 0 | 0.39 | 0.22 | 0.10 |
| RASK-4 | 0.18 | 0.08 | 0.03 | 0.19 | 0.10 | 0.04 |
| None | 0.64 | 0.27 | 0.10 | 0.70 | 0.41 | 0.15 |

### EXAMPLE 5

Reactivity of a Monoclonal Antibody (RASK-3) with Thyroid Carcinoma Tissues (by the ABC Method):

A monoclonal antibody (RASK-3) was examined for its reactivity with 10% formalin-fixed thyroid carcinoma sections from 50 patients by the above-mentioned ABC method. The results obtained are shown in Table 5. As shown in Table 5, the antibody showed totally strong positive reaction with section portions pathomorphologically diagnosable as carcinomatous in 40 cases and, in 10 cases, it showed partly strong positive reaction with such section portions. On the other hand, with non-cancerous portions, it showed partly positive reaction in 3 cases and partly weakly positive reaction in 25 cases; it gave totally negative results in 22 cases. Other monoclonal antibodies gave similar results.

## TABLE 5

| | Reactivity with Antibody | | | |
|---|---|---|---|---|
| Sample | Totally Positive | Partly Positive | Partly Weakly Positive | Negative |
| Cancerous Portion of Thyroid Gland | 40/50 | 10/50 | 0/50 | 0/50 |
| Non-cancerous Porttion of Thyroid Gland | 0/50 | 3/50 | 25/50 | 22/50 |

## EXAMPLE 6

Reactivity of the Monoclonal Antibodies with Stomach Carcinoma (As estimated by ABC Method):

The monoclonal antibodies according to the invention were examined for their reactivity with 10% formalin-fixed stomach carcinoma sections from 42 patients by the ABC method. The results obtained with RASK-3 are shown in Table 6 and Table 7. As shown in Table 6, with section portions pathomorphologically diagnosable as carcinomatous, RASK-3 showed totally strong positive reaction in 31 cases, partly but strongly positive reacton in 6 cases, and totally negative reaction in 5 cases. On the other hand, with normal epithelial cells, the reaction was partly and weakly positive in 3 cases and, in the remaining 39 cases, the reaction was negative. The frequencies of positive responses of stomach carcinoma sections as investigated and classified by histological type are shown in Table 7. In all the 3 papillary adenocarcinoma cases and all the 12 well differentiated tubular adenocarcinoma case, the response was totally and strongly positive. In 9 out of 11 moderately differentiated tubular adenocarcinoma cases, the response was totally positive. As regards poorly differentiated adenocarcinoma cases, the response was totally and strongly positive in 5 out of 12 cases, and partly but strongly positive in 6 cases. In both the two cases of mucinous adenocarcinoma, the response was positive, while the response was negative in both of the two signet ring cell carcinoma cases.

## TABLE 6

| Sample | Totally Positive | Partly Positive | Partly Weakly Positive | Negative |
|---|---|---|---|---|
| Reactivity with Antibody | | | | |
| Stomach Carcinoma Portion | 31/42 | 6/42 | 0/42 | 5/42 |
| Normal Epithelial Cells | 0/42 | 0/42 | 3/42 | 39/42 |

## TABLE 7

| Sample | Frequency of Positive Responses of Stomach Carcinomas as Classified by Histological Type |
|---|---|
| Papillary Adenocarcinoma | 3/3 |
| Tubular Adenocarcinoma | |
|     Well Differentiated | 12/12 |
|     Moderately Differentiated | 9/11 |
| Poorly Differentiated Adenocarcinoma | 11/12 (in 6 of which results were partly positive) |
| Mucinous Adenocarcinoma | 2/2 |
| Signet Ring Cell Carcinoma | 0/2 |

EXAMPLE 7

Reactivity of the Monoclonal Antibodies with Colon Carcinoma, Uterine Carcinoma and Breast Carcinoma (as estimated by the ABC Method):

The above monoclonal antibodies were evaluated for their reactivity with 10% formalin-fixed colon, uterine and breast carcinoma sections by the ABC method. All the antibodies strongly reacted with colon, uterine and breast carcinoma tissues, while they gave weakly positive or negative results with normal tissues.

## Claims

1. A monoclonal antibody which belongs to the class IgG and is reactive with the Ki-ras-oncogene product.

2. The monoclonal antibody of claim 1, wherein said Ki-ras oncogene product is Ki-p21.

3. The monoclonal antibody of claim 1 as specified by the designation RASK-1, RASK-2, RASK-3, or RASK-4.

4. A method of detecting cancer comprising (a) contacting a patient-derived tissue sample with a monoclonal antibody that is reactive with the Ki-ras oncogene product and (b) assessing the reactivity of the monoclonal antibody to the tissue sample.

5. The method of claim 4, wherein the cancer is human thyroid carcinoma, human stomach carcinoma, human breast carcinoma, human uterine carcinoma or human colon carcinoma.

6. The method of claim 4 in which the reactivity is assessed using indirect immunofluoresence assay, enzyme-linked immunosorbent assay, avidin-biotin complex enzyme immunoassay or immunoblotting.

7. A hybridoma which is capable of producing a monoclonal antibody reactive with the Ki-ras oncogene product.

8. The hybridoma of claim 7 as specified by the deposition Nos. ECACC 86091101, ECACC 86091102, ECACC 86091103 or ECACC 86091104.

9. An immunoassay reagent for use in the detection of cancer comprising at least one monoclonal antibody of claims 1 to 3.

10. The reagent of claim 9 wherein the monoclonal antibody is produced by a hybridoma which is deposited with the European Collection of Animal Cell Cultures under ECACC accession numbers 86091101, 86091102, 86091103 or 86091104.